# EUROPEAN PATENT APPLICATION

(11) **EP 4 331 587 A1**
(43) Date of publication of application: **06.03.2024**
(21) Application number: 22796144.8
(22) Date of filing: 27.04.2022
(51) Int. Cl.: A61K 31/5377, A61P 3/00, A61P 3/04, A61P 1/16, A61P 35/00

(54) **COMPOSITION FOR PREVENTING OR TREATING METABOLIC DISEASES, CONTAINING TRICYCLO DERIVATIVE COMPOUND**

(30) Priority: 30.04.2021 KR 20210056690
(71) Applicant: Oncocross Co., Ltd., Seoul 04168 (KR); Jeil Pharmaceutical Co., Ltd., Seoul 06543 (KR)
(72) Inventor: KIM, Yi Rang, Sejong 30064 (KR); PARK, Sang Yun, Seoul 08329 (KR); YANG, Ga Eul, Anyang-si Gyeonggi-do 13974 (KR)
(74) Representative: Isarpatent
(86) International application number: PCT/KR2022/006041
(87) International publication number: WO 2022/231312

(57) **Abstract**

The present invention relates to a composition for preventing or treating metabolic diseases, containing 10-ethoxy-8-(morpholinomethyl)-2,3,4,6-tetrahydrobenzo[h][1,6]naphthyridin-5(1H)-one, a pharmaceutically acceptable salt thereof, a hydrate thereof, or a salt hydrate thereof as an active ingredient. Due to an adipocyte differentiation inhibitory effect, a fat accumulation inhibitory effect, an NAFLD inhibitory effect, and an NASH-induced fibrosis inhibitory effect of the composition, it is possible to prevent or treat metabolic diseases including nonalcoholic steatohepatitis (NASH), NASH-associated liver fibrosis, nonalcoholic fatty liver (NAFL), and NAFLD-associated liver fibrosis, fatty liver, and obesity.

## Description

### [Technical Field]

The present invention relates to a composition for preventing or treating metabolic diseases, in which the composition contains a tricyclo derivative compound of 10-ethoxy-8-(morpholinomethyl)-2,3,4,6-tetrahydrobenzo[h][1,6]naphthyridin-5(1H)-one, a pharmaceutically acceptable salt thereof, a hydrate thereof, or a salt hydrate thereof.

### [Background Art]

Recently, with economic development and changes in eating habits, the incidence of metabolic diseases (metabolic syndrome), which includes various diseases such as obesity, hyperlipidemia, hypertension, arteriosclerosis, hyperinsulinemia, diabetes, and liver disease, is rapidly increasing. These diseases may occur individually, but are generally closely related to each other and most often occur together with various symptoms. Since metabolic diseases were named by Reaven GM in 1988, the metabolic diseases have become common health problem in primary medical care. The prevalence of metabolic diseases in the primary medical care is known to be approximately 30%, and the prevalence increases with age and severity of obesity. The medical importance of the metabolic disease is revealed from the fact that the mortality rate of cardiovascular disease is higher in cases with metabolic disease than in cases without metabolic disease. The obese population is gradually increasing in Korea as well, and the prevalence of cardiovascular diseases such as hypertension, diabetes, and hyperlipidemia is rapidly increasing. Considering that the mortality rate from cardiovascular diseases ranks second in men and women, recently, efforts to prevent and treat metabolic diseases are continuing.

Obesity is widely known to cause chronic diseases such as fatty liver, hypertension, diabetes, and cardiovascular disease, and the number of obese people is rapidly increasing, emerging as a serious social issue. Anti-obesity drugs sold at home and abroad include Xenical, which is mainly made of orlistat, which has been approved by the U.S. FDA. However, Xenical, which inhibits lipase action, has gastrointestinal side effects such as steatorrhea, gas production, and reduced absorption of fat-soluble vitamins. Adipogenesis and fat accumulation are caused by metabolic diseases (metabolic syndrome) or autoimmune reactions, which cause excessive fat metabolism and an increase in adipose tissue. Dietary or drug treatments are being used as treatments to alleviate the symptoms of fat accumulation in various diseases, but to date, a method to fundamentally treat fat accumulation has not been developed.

Non-alcoholic fatty liver disease (NAFLD) is defined as the accumulation of fatty acids in the form of triglycerides in hepatic parenchymal cells by 5% or more, rather than liver damage caused by alcohol. Pathologically, the NAFLD is classified as simple steatosis and steatohepatitis accompanied by inflammation. Non-alcoholic steatohepatitis (NASH) is a disease that occurs during the worsening process of the NAFLD. As fat accumulates within liver cells, hepatocyte degeneration/necrosis occurs and the resulting inflammation and liver fibrosis develops into cirrhosis and hepatocellular carcinoma. Accordingly, it is recognized as a serious disease worldwide. It has been suggested that the NASH plays an important role in causing direct liver damage along with intrahepatic fat accumulation due to excessive free fatty acid (FFA) in the body. In the case of obesity and insulin resistance, the influx of FFA into the liver increases, and the FFA accumulates fat in the liver through β-oxidation or esterification of triglyceride (TG). In addition, direct liver damage occurs due to an increase in oxidative stress caused by excessive FFA itself and activation of the inflammatory signaling process, and the TG accumulates in the liver as a protective mechanism against this. This leads to hepatocyte death and insufficient repair, ultimately leading to the NASH, a serious lesion that causes liver fibrosis and disease progression (Ong JP et al., Obesity Surgery volume 15, pages 310-315, 2005). The NASH is divided into primary and secondary depending on the cause. It is known that primary is caused by hyperlipidemia, diabetes, obesity, or the like, which are characteristics of metabolic syndrome (Szczepaniak LS et al., 2005), and that secondary is caused by nutritional causes (rapid weight loss, starvation, and intestinal bypass surgery), various drugs, toxic substances (poisonous mushrooms, and bacterial toxins), metabolic causes, and other factors. The incidence of the NASH related to diabetes and obesity, which are important characteristics of metabolic syndrome, which is a primary factor, is known to occur in approximately 50% of diabetic patients, approximately 76% of obese patients, and almost all of the obese diabetic patients (Gupte P *et al.,* 2004). In addition, when biopsies were performed on diabetic and obese patients with increased levels of alanine aminotransferase (ALT), the incidence of steatohepatitis was found to be 18 to 36% (Braillon A *et al.,* 1985).

The NASH has been considered a benign disease that does not progress, but it has been found to show histological patterns of inflammation and liver fibrosis similar to alcoholic hepatitis even in non-drinkers, and is known to be a disease with a poor prognosis. In particular, metabolic syndromes due to obesity, diabetes, or the like have been drawing attention in recent years. It is becoming a common view that the NASH is one of such syndromes. However, the mechanism remains unclear, and effective treatment methods and/or treatment agents have not been established. To date, there is no established treatment for the NASH, because the NASH is associated with various factors such as diabetes, obesity, coronary artery disease, and lifestyle. The effectiveness of several diabetes or obesity treatment drug administration for fatty liver disease has been reported. Orlistat, an oral anti-obesity drug, has been reported to induce liver histological improvement in patients with steatohepatitis (Hussein *et al.,* 2007). In addition, there is a report that metformin reduced liver enzyme levels in blood, liver necrotic inflammation, and fibrosis in patients with the NASH without diabetes (Bugianesi *et al.*, 2005). In addition, it has been reported that thiazolidinedione (TZD) drugs, which are agonists of peroxisome proliferatoractivated receptor (PPAR), inhibit fat accumulation in the liver and muscles and exhibit direct anti-fibrotic action on the liver in animal models of the NASH (Galli A *et al.,* 2002). However, there is currently no composition that may be approved as a drug treatment for the NASH, and there is an urgent need to develop new therapeutic agents for the treatment or prevention of the disease.

### [Disclosure]

### [Technical Problem]

An aspect of the present invention is directed to providing a pharmaceutical composition for preventing or treating metabolic diseases.

In addition, an aspect of the present invention is directed to providing a use for preventing or treating metabolic diseases.

In addition, an aspect of the present invention is directed to providing a method for preventing or treating metabolic diseases.

### [Technical Solution]

An embodiment of the present invention provides a pharmaceutical composition for preventing or treating metabolic diseases, in which the pharmaceutical composition contains 10-ethoxy-8-(morpholinomethyl)-2,3,4,6-tetrahydrobenzo[h][1,6]naphthyridin-5(1H)-one, a pharmaceutically acceptable salt thereof, a hydrate thereof, or a salt hydrate thereof as an active ingredient.

In addition, an embodiment of the present invention provides a use of 10-ethoxy-8-(morpholinomethyl)-2,3,4,6-tetrahydrobenzo[h][1,6]naphthyridin-5(1H)-one, a pharmaceutically acceptable salt thereof, a hydrate thereof, or a salt hydrate thereof, for preventing or treating metabolic diseases.

In addition, an embodiment of the present invention provides a method for preventing or treating metabolic diseases, in which the method includes administering 10-ethoxy-8-(morpholinomethyl)-2,3,4,6-tetrahydrobenzo[h][1,6]naphthyridin-5(1H)-one, a pharmaceutically acceptable salt thereof, a hydrate thereof, or a salt hydrate thereof in a pharmaceutically effective amount to a subject suffering from the metabolic diseases.

### [Advantageous Effects]

10-ethoxy-8-(morpholinomethyl)-2,3,4,6-tetrahydrobenzo[h][1,6]naphthyridin-5(1H)-one, a pharmaceutically acceptable salt thereof, a hydrate thereof, or a salt hydrate thereof of an embodiment of the present invention inhibits fat accumulation by inhibiting differentiation into adipocytes, inhibits NASH through inhibition of PARylation, and significantly decreases fibrosis induced by the NASH. Accordingly, the aforementioned ingredients can be useful for the prevention or treatment of metabolic diseases including non-alcoholic steatohepatitis (NASH), non-alcoholic fatty liver (NAFL), NAFLD-related liver fibrosis, fatty liver, and obesity.

### [Description of Drawings]

FIG. 1 is a diagram identifying the cytotoxicity of JPI-289 in various cell lines by MTT analysis.
FIG. 2 is a diagram identifying the cytotoxicity of JPI-289 in 3T3L1 cell line by MTT analysis.
FIG. 3 is a diagram identifying the effect of JPI-289 on inhibiting intracellular fat accumulation by inhibiting adipocyte differentiation in the 3T3L1 cell line.
FIG. 4 is a diagram identifying the minimum concentration showing the effect of JPI-289 on inhibiting intracellular fat accumulation by inhibiting adipocyte differentiation in the 3T3L1 cell line.
FIG. 5 is a diagram identifying the inhibitory effect of JPI-289 on intracellular fat accumulation in HepG2 cell line.
FIG. 6 is a diagram comparing the inhibitory effects of other PARP inhibitors, 5-AIQ (10 uM), PJ34 (10 uM), and olaparib (5 uM), and JPI-289 (0.25 to 2.5 uM) on fat accumulation.
FIG. 7 is a diagram comparing the HIF-1 reduction effects of JPI-289 (JPI) and PJ-34 (PJ) after creating an NASH environment in the Hep3B cell line:
   JPI: JPI-289; and
   PJ: PJ34.
FIG. 8 is a diagram comparing the HIF-1 reduction effects of JPI-289 and INT-747 after creating an NASH environment in the Hep3B cell line:
   JPI: JPI-289; and
   INT: INT-747.
FIG. 9 is a diagram identifying the level of HIF-1α expression by administration of JPI-289, PJ34, or INT-747 in a high-fat diet mouse model:
   HFD: high-fat diet;
   JPI: JPI-289 50 mpk; and
   INT: INT-747.
FIG. 10 is a diagram comparing the inhibitory effect of TGF-beta2 (downstream signal of HIF-1) by JPI-289 and INT-747 at the RNA level:
   JPI: JPI-289; and
   INT: INT-747.
FIG. 11 is a schematic diagram showing the effect mechanism of JPI-289 in treating NASH and NASH-induced fibrosis:
   JPI: JPI-289.
FIG. 12 is a diagram showing a schedule for drug administration to a high-fat diet mouse model:
   LFD: low-fat diet;
   HFD: high-fat diet;
   JPI: JPI-289; and
   INT: INT-747.
FIG. 13 is a diagram identifying the change in body weight of the JPI-289 (JPI) administration group in a high-fat diet mouse model:
   LFD: low-fat diet;
   HFD: high-fat diet;
   JPI: JPI-289; and
   INT: INT-747.
FIG. 14 is a diagram identifying changes in liver weight and size of the JPI-289 (JPI) administration group in a high-fat diet mouse model:
   LFD: low-fat diet;
   HFD: high-fat diet;
   JPI: JPI-289; and
   INT: INT-747.
FIG. 15 is a schematic diagram showing the time of disease onset in the STAM^{™} mouse model.
FIG. 16 is a diagram showing the administration schedule of JPI-289 in the STAM^{™} mouse model:
   STAM^{™}: STAM^{™} mouse model;
   HFD: high-fat diet;
   Oral: oral administration;
   QD: once daily; and
   J-001: JPI-289.
FIG. 17 is a diagram identifying the effect of reducing liver fibrosis area by short-term administration of JPI-289 in the STAM^{™} mouse model:
   low: 10 mg/kg;
   high: 50 mg/kg; and
   J-001: JPI-289.
FIG. 18 is a diagram identifying the fatty liver reduction effect by short-term administration of JPI-289 in the STAM^{™} mouse model with NAFLD score and ballooning score:
   J-001: JPI-289;
   low: 10 mg/kg; and
   high: 50 mg/kg.
FIG. 19 is a diagram showing the administration schedule of JPI-289 in the STAM^{™} mouse model:
   STAM^{™}: STAM^{™} mouse model;
   HFD: high-fat diet;
   Oral: oral administration;
   QD: once daily; and
   J-001: JPI-289.
FIG. 20 is a diagram identifying the effect of reducing liver fibrosis area by long-term administration of JPI-289 in the STAM^{™} mouse model:
   J-001 low: JPI-289 10 mg/kg.
FIG. 21 is a diagram identifying the fatty liver reduction effect by long-term administration of JPI-289 in the STAM^{™} mouse model with NAFLD score and ballooning score:
   J-001 low: JPI-289 10 mg/kg.

### [Modes of the Invention]

Hereinafter, the present invention will be described in detail by way of embodiments of the present invention with reference to the accompanying drawings. However, the following examples are provided by way of illustration of the present invention. When it is determined that the specific description of known techniques or configuration well known to those skilled in the art unnecessarily obscures the gist of the present invention, the detailed description therefor may be omitted, and the present invention is not limited thereto. The present invention allows various modifications and applications within the description of the claims to be described later and the scope of equivalents interpreted therefrom.

Further, terminologies used herein are terms used to properly represent preferred embodiments of the present invention. It may vary depending on the intent of users or operators, or custom in the art to which the present invention belongs. Accordingly, the definitions of these terms should be based on the contents throughout this specification. In the entire specification, when a part is referred to as "comprising" a component, it means that it may further include other components without excluding other components unless specifically described otherwise.

Unless otherwise defined, all the technical terms used herein are used with the same meaning as commonly understood by a person skilled in the art in the field related to the present invention. In addition, preferred methods or samples are described herein, but those similar or equivalent thereto are incorporated in the scope of the present invention. The contents of all the publications disclosed as references herein are incorporated in the present invention.

In an aspect, an embodiment of the present invention relates to a pharmaceutical composition for preventing or treating metabolic diseases, in which the pharmaceutical composition contains, as an active ingredient, a novel crystalline acid addition salt of a tricyclo derivative compound represented by the following Formula 1 or a hydrate thereof:

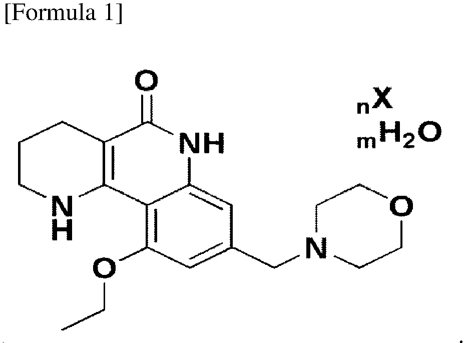

In Formula 1 above, n or m is an integer from 0 to 3; and X is a pharmaceutically acceptable inorganic acid or organic acid.

In an embodiment, the n or m in Formula 1 above may be an integer from 0 to 3; and the X may be one type selected from the group consisting of a hydrochloric acid, a benzene sulfonic acid, a maleic acid, a dimethane sulfonic acid, bis[7,7-dimethyl-2-oxobycyclo[2,2,1]heptane-1-yl)methanesulfonic acid], a tartaric acid, a 2,6-dioxo-1,2,3,6-tetrahydropyrimidine-4-carboxylic acid, an adipic acid, a nitrous acid (dinitric acid), a fumaric acid, a (S)-2-aminosuccinic acid, a 2-hydroxypropane-1,2,3-tricarboxylic acid, a cyclohexylsulphamic acid, a sulfuric acid, a succinic acid, a formic acid, a glutamic acid, and a diphosphoric acid.

Preferably, an embodiment of the present invention relates to a pharmaceutical composition for preventing or treating metabolic diseases, in which the pharmaceutical composition contains 10-ethoxy-8-(morpholinomethyl)-2,3,4,6-tetrahydrobenzo[h][1,6]naphthyridin-5(1H)-one, a pharmaceutically acceptable salt thereof, a hydrate thereof, or a salt hydrate thereof as an active ingredient.

In an aspect, an embodiment of the present invention relates to a pharmaceutical composition for preventing or treating metabolic diseases, in which the pharmaceutical composition contains 10-ethoxy-8-(morpholinomethyl)-2,3,4,6-tetrahydrobenzo[h][1,6]naphthyridin-5(1H)-one dihydrochloride dihydrate as an active ingredient.

In an embodiment, the 10-ethoxy-8-(morpholinomethyl)-2,3,4,6-tetrahydrobenzo[h][1,6]naphthyridin-5(1H)-one dihydrochloride dihydrate may be represented by Formula 2 below:

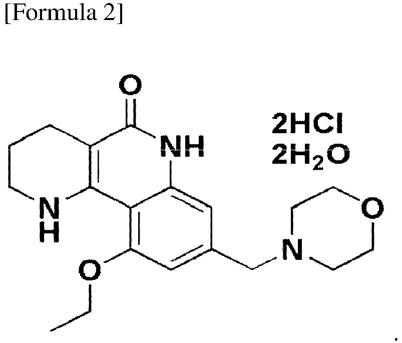

In an embodiment of the present invention, the 10-ethoxy-8-(morpholinomethyl)-2,3,4,6-tetrahydrobenzo[h] [1,6]naphthyridin-5(1H)-one dihydrochloride dihydrate of Formula 2 above was named and used as JPI-289.

Preferably, for the 10-ethoxy-8-(morpholinomethyl)-2,3,4,6-tetrahydrobenzo[h][1,6]naphthyridin-5(1H)-one dihydrochloride dihydrate, specific peaks expressed in 2θ may appear at 6.59°, 7.74°, 8.42°, 10.15°, 12.52°, 23.30° and 25.30° in the X-ray diffraction pattern of Cu target radiation.

In an embodiment, the metabolic disease may be any one selected from the group consisting of obesity, cirrhosis, and metabolic liver disease, and the metabolic liver disease may be fatty liver or non-alcoholic fatty liver disease (NAFLD). The NAFLD may be any one or more selected from the group consisting of non-alcoholic steatohepatitis (NASH), non-alcoholic fatty liver (NAFL), NAFLD-related liver fibrosis, NASH-related liver fibrosis, NAFLD-related liver cancer (Hepatoma), and NASH-related liver cancer (Hepatocellular carcinoma).

In an embodiment, the composition of an embodiment of the present invention may inhibit PARylation, increase TIPARP (TCDD-inducible poly [ADP-ribose] polymerase), and inhibit or decrease the expression of HIF-1α (hypoxia inducible factor-1α), PARP1 (Poly [ADP-ribose] polymerase 1), vimentin, and TGF-beta2.

In an embodiment, the composition of an embodiment of the present invention may inhibit and decrease fat accumulation by inhibiting differentiation into adipocytes, and may inhibit or decrease fat accumulation in the liver.

In an embodiment, the composition of an embodiment of the present invention is capable of inhibiting or reducing fibrosis, inhibiting fibrosis of the liver, and inhibiting or reducing fibrosis induced by NASH.

In an embodiment, the 10-ethoxy-8-(morpholinomethyl)-2,3,4,6-tetrahydrobenzo[h][1,6]naphthyridin-5(1H)-one dihydrochloride dihydrate of an embodiment of the present invention may be administered in doses of 0.0001 to 1,000 mg/kg, preferably 0.01 to 500 mg/kg, once or several times a day.

When the composition of an embodiment of the present invention is used as a medicine, the pharmaceutical composition may be prepared and administered in various oral or parenteral dosage forms as described below during clinical administration, but is not limited thereto.

Formulations for oral administration may include, for example, tablets, pills, hard/soft capsules, solutions, suspensions, emulsifiers, syrups, granules, elixirs, troches, etc. In addition to the active ingredients, these formulations may contain diluents (for example, lactose, dextrose, sucrose, mannitol, sorbitol, cellulose and/or glycine), and lubricants (for example, silica, talc, stearic acid and its magnesium or calcium salts and/or polyethylene glycol). Oral solid preparations may also contain binding agents such as magnesium aluminum silicate, starch paste, gelatin, methylcellulose, sodium carboxymethylcellulose and/or polyvinylpyrrolidine, and in some cases, may contain disintegration agents such as starch, agar, alginic acid or its sodium salt or similar mixture and/or absorbent, coloring, flavor and sweetener. The preparations may be prepared by general mixing, granulation or coating methods.

The pharmaceutical composition may be administered parenterally, and parenteral administration is administered through a method of infusing a subcutaneous injection, an intravenous injection, an intravenous drip injection, an intramuscular injection, or an intrathoracic injection.

In this connection, in order to prepare a formulation for parenteral administration, the composition is mixed with water along with a stabilizer or buffer to prepare a solution or suspension, which may be prepared in an ampoule or vial unit dosage form. The composition may be sterilized and/or may contain adjuvants such as antiseptics, stabilizers, hydrating agents, or emulsification accelerators, salts and/or buffers for adjusting osmotic pressure, and other therapeutically useful substances.

The dosage for the human body of the pharmaceutical composition of an embodiment of the present invention may vary depending on age, body weight, gender, dosage form, health condition, and disease severity of a patient. The dosage may be divided and administered through oral or parenteral routes at regular intervals several times a day, preferably once to three times a day, depending on the decision of a doctor or pharmacist, preferably in an amount of 0.0001 to 1000 mg/kg, preferably 0.01 to 500 mg/kg.

The pharmaceutical composition of an embodiment of the present invention may further include known therapeutic agents for metabolic diseases in addition to the compounds as active ingredients, and may be used in combination with other known treatments for the treatment of these diseases.

As used herein, the term "prevention" means all actions for inhibiting or delaying the onset, spread, and recurrence of metabolic diseases by administration of the pharmaceutical composition according to an embodiment of the present invention, and the term "treatment" means all actions involved in alleviating or beneficially changing symptoms of metabolic diseases by administration of the composition of an embodiment of the present invention. Those skilled in the art can appreciate the exact criteria of the disease on which the compositions herein have effects and determine the extent of improvement, enhancement, and treatment with reference to the data presented by the Korean Academy of Medical Sciences, etc.

As used herein, the term "active ingredient" means an amount effective to prevent or treat metabolic diseases, and is administered in a therapeutically effective amount of the composition of an embodiment of the present invention. The therapeutically effective amount of the composition of an embodiment of the present invention may vary depending on various factors, for example, administration method, target site, the patient's condition, and the like. Accordingly, the dosage when used in the human body should be determined in appropriate amounts in consideration of safety and efficacy. It is also possible to estimate the amount used in humans from the effective amount determined by animal experiments. These matters to be considered in determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of an embodiment of the present invention is administered in a pharmaceutically effective amount. The level of an effective dosage may be determined by parameters including a health status of a patient, the kind of metabolic diseases, the cause of metabolic diseases, severity, the activity of a drug, sensitivity to a drug, an administration method, administration time, an administration route and a release rate, duration of treatment, formulated or co-used drugs, and other parameters well known in medical fields. The composition of an embodiment of the present invention may be administered as an individual therapeutic agent or in combination with other therapeutic agents. It may be administered sequentially or simultaneously with a conventional therapeutic agent or administered in a single or multiple-dose regime. In consideration of all of the above parameters, it is important to administer such a dose to obtain a maximum effect with a minimal amount without a side effect and the dose may be easily determined by those skilled in the art.

The pharmaceutical composition of an embodiment of the present invention may effectively prevent or treat the above diseases by administering the same to all animals, including monkeys, cows, horses, sheep, pigs, chickens, turkeys, quails, cats, dogs, mice, rats, rabbits or guinea pigs, including humans who have or may develop the metabolic disease. The pharmaceutical composition of an embodiment of the present invention may be administered in combination with existing therapeutic agents.

In an aspect, an embodiment of the present invention relates to a use of 10-ethoxy-8-(morpholinomethyl)-2,3,4,6-tetrahydrobenzo[h][1,6]naphthyridin-5(1H)-one, a pharmaceutically acceptable salt thereof, a hydrate thereof, or a salt hydrate thereof, for preventing or treating metabolic diseases.

In an aspect, an embodiment of the present invention relates to a method for preventing or treating metabolic diseases, in which the method includes administering 10-ethoxy-8-(morpholinomethyl)-2,3,4,6-tetrahydrobenzo[h][1,6]naphthyridin-5(1H)-one, a pharmaceutically acceptable salt thereof, a hydrate thereof, or a salt hydrate thereof in a pharmaceutically effective amount to a subject suffering from the metabolic diseases.

### [Modes of Carrying Out the Invention]

Hereinafter, the present invention will be described in more detail with reference to the following Examples. However, the following Examples are only intended to embody the contents of the present invention, and the present invention is not limited thereto.

### Example 1. Identification of Cytotoxicity of JPI-289

### 1-1. Identification of JPI-289 toxicity in multiple cell lines

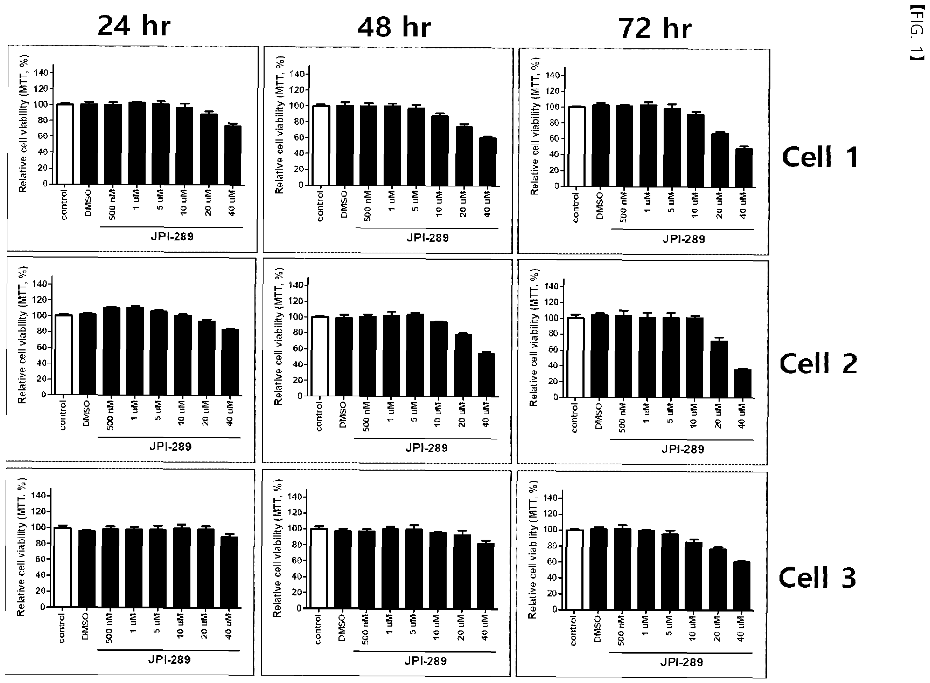

To identify the toxicity of JPI-289 to cells, MTT analysis was performed using three cell lines: MCF7, PC3, and HL60 (indicated as Cell 1 to Cell 3 in FIG. 1, respectively). Specifically, each cell line was treated with a control group, a DMSO control group (vehicle control), and JPI-289 (10-ethoxy-8-(morpholinomethyl)-2,3,4,6-tetrahydrobenzo[h][1,6]naphthyridin-5(1H)-one dihydrochloride dihydrate) at concentrations of 0.5, 1, 5, 10, 20, and 40 uM, and then treated with MTT tetrazolium after 24, 48, and 72 hours. Then, the cell viability was measured by forming formazan crystals, dissolving the same using DMSO (dimethyl sulfoxide), and measuring the absorbance using an ELISA reader.

As a result, it was identified that JPI-289 was not toxic to cells for 72 hours up to a concentration of 10 uM, and was identified to be toxic at concentrations of 20 and 40 uM (FIG. 1).

### 1-2. Identification of JPI-289 toxicity in preadipocyte cell lines

The 3T3L1 cell line, a preadipocyte cell line, was dispensed in a 96-well plate, and treated with JPI-289 at a concentration of 0.05 to 5 uM for 24, 48, or 72 hours, and MTT analysis was performed.

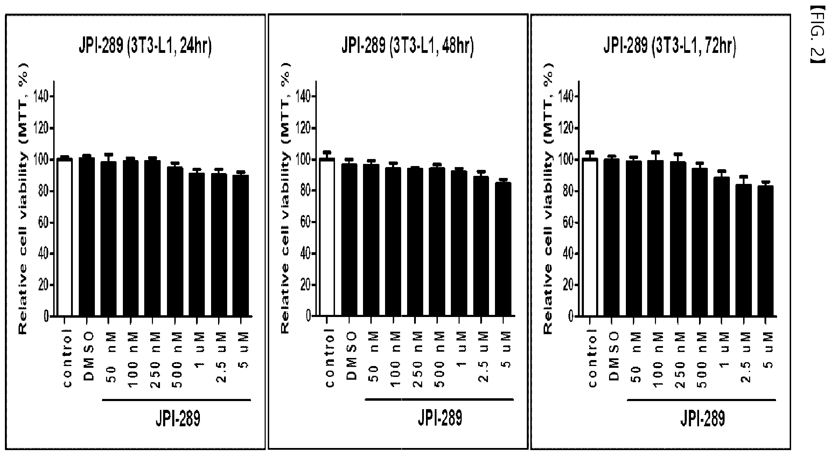

As a result, it was shown to have no significant effect in 3T3L1 (FIG. 2).

### Example 2. Inhibitory Effect of JPI-289 on Intracellular Fat Accumulation

### 2-1. Identification of inhibitory effect on adipocyte differentiation of preadipocyte cell lines

To induce differentiation of mouse preadipocyte cell line 3T3L1 into adipocytes, DMEM medium containing 10% FBS was treated with insulin, dexamethasone, and isobutylmetylxanthin (IBMX). JPI-289 was treated with a differentiation medium from the start day of inducing differentiation into adipocytes to the end of differentiation. At the end of differentiation, cells were fixed and differentiated adipocytes were stained red using Oil-Red O. The degree of staining was recorded in a photograph, Oil Red was dissolved, and the O.D. value was measured using an ELISA reader.

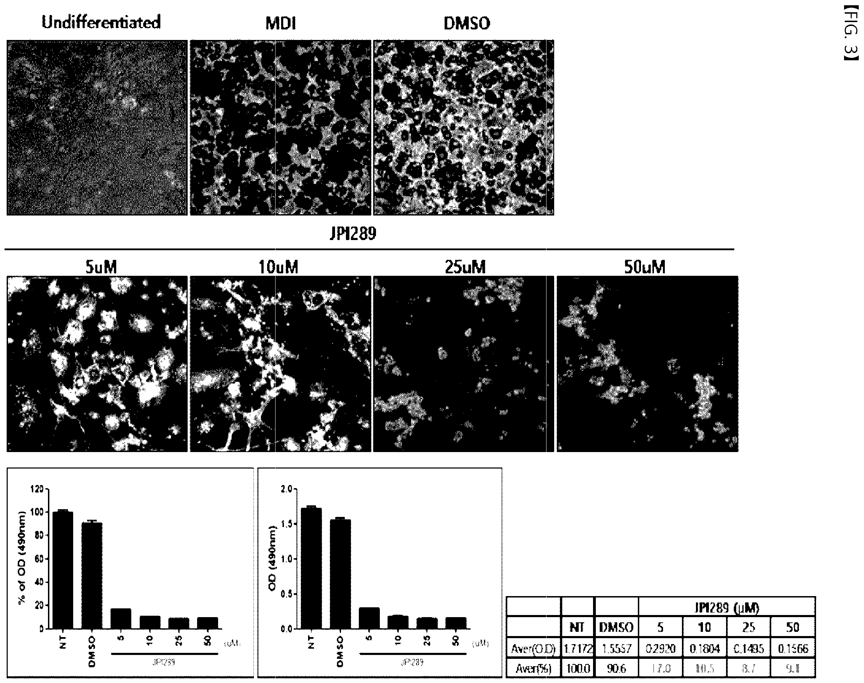

As a result, it was identified that adipocytes were not able to be observed in the group that did not induce differentiation, and that differentiation into adipocytes was well induced in the group containing only differentiation medium. In addition, in the group treated with differentiation medium and JPI-289, differentiation into adipocytes was inhibited depending on the concentration at a concentration of 5 uM or higher, and fat accumulation in 3T3L1 cells was significantly decreased (FIG. 3).

### 2-2. Identification of concentration that inhibits adipocyte differentiation

To identify the inhibitory effect of JPI-289 at a concentration of 5 uM or less on adipocyte differentiation, a re-experiment was performed on the 3T3L1 cell line. Specifically, JPI-289s at concentrations of 0.05, 0.1, 0.25, 0.5, 1, and 2.5 uM were treated together with the differentiation culture medium, respectively, to induce differentiation into adipocytes, and the differentiation into adipocytes and fat accumulation were identified by staining using Oil-Red O.

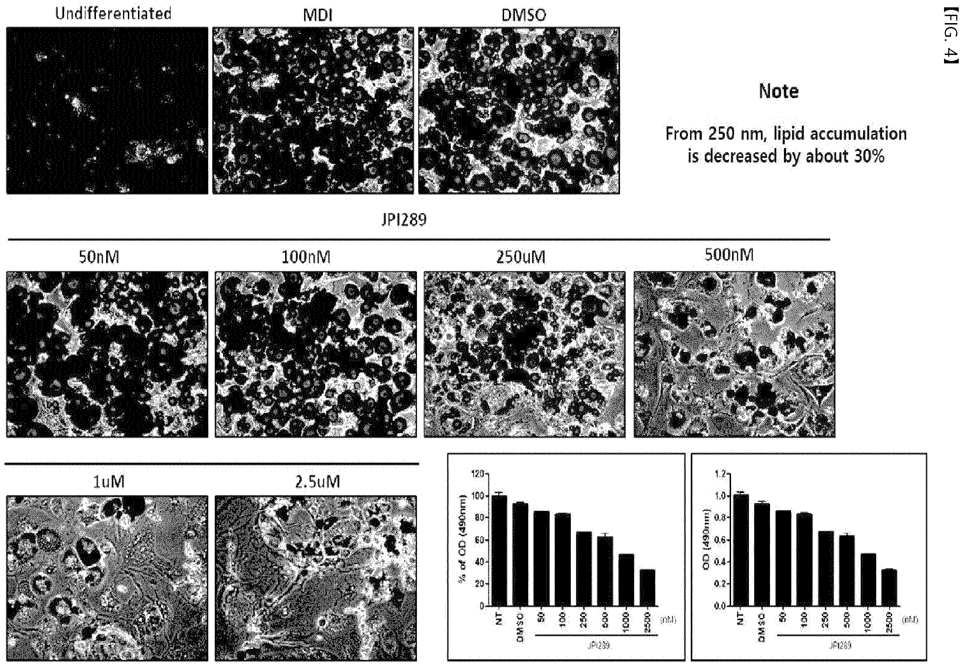

As a result, it was identified that in the group treated with JPI-289, differentiation into adipocytes was inhibited depending on the concentration. Even at the lowest concentration of 50 nM (0.05 uM), differentiation inhibitory effect was shown by about 15%, and fat accumulation was significantly decreased starting from 250 nM (0.25 uM) (about 37%). Through the experiment, it was identified that the inhibitory efficacy on adipocyte differentiation increased as the concentration of JPI-289 increased (FIG. 4).

### 2-3. Identification of fat accumulation inhibitory effect in liver cancer cell lines

By treating the HepG2 cell line, a liver cancer cell line, with oleic acid, fat accumulation was induced into cells, and simultaneously, JPI-289 was treated together to identify the effect of JPI-289 on inhibiting fat accumulation using the Oil-Red O staining method. Herein, INT-747, a drug that succeeded in phase 3 clinical trials for NASH indication, was treated as a positive control group.

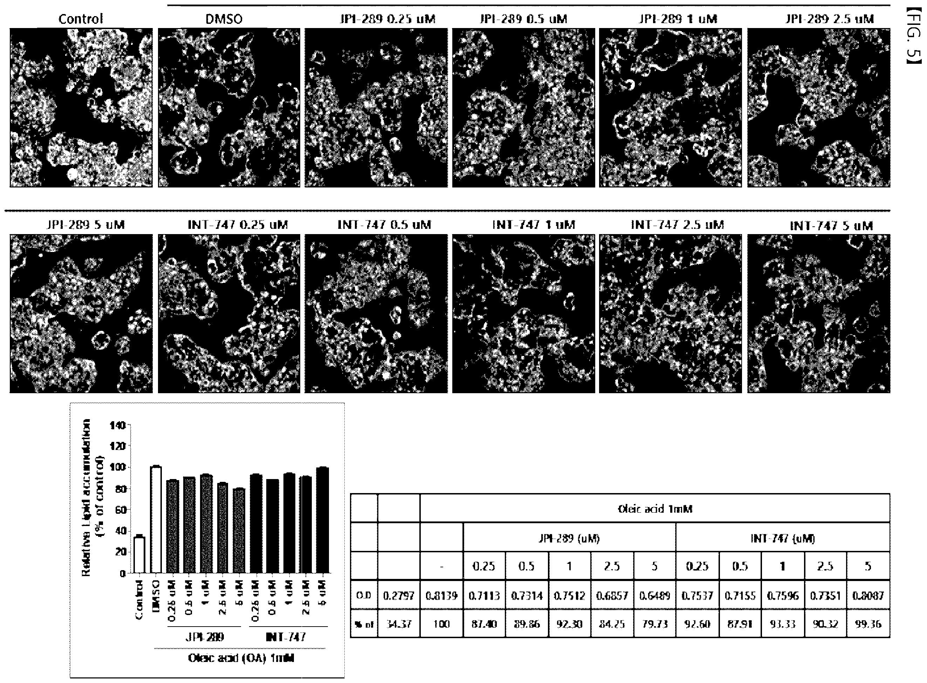

As a result, INT-747, a positive control group, showed a fat accumulation inhibitory effect of about 13%, while JPI-289 showed a significant fat accumulation inhibitory effect of about 21% (FIG. 5). It was identified that JPI-289 was more effective at high concentrations, whereas INT-747 was rather less effective in inhibiting fat accumulation at high concentrations.

### Example 3. Comparison of Fat Accumulation Inhibitory Effects with Other PARP Inhibitor Drugs

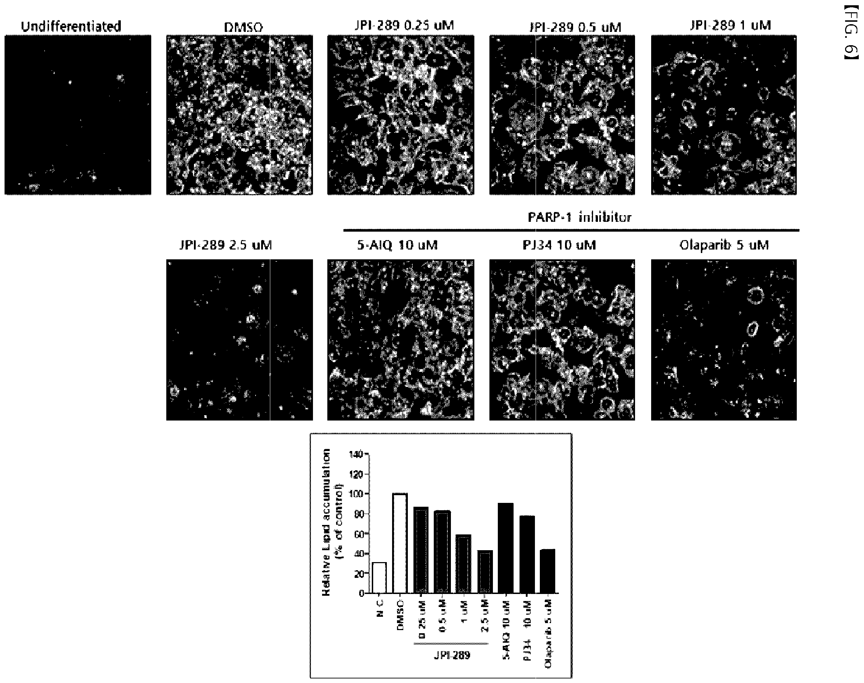

The fat accumulation inhibitory effects of other PARP inhibitors, 5-AIQ (10 uM), PJ34 (10 uM), and olaparib (5 uM), and JPI-289 (0.25 to 2.5 uM) were compared in the mouse preadipocyte cell line 3T3L1 by the method of Example 2 above. As a result, JPI-289 of an embodiment of the present invention showed a significantly superior fat accumulation inhibitory effect at all concentrations significantly lower than 5-AIQ and PJ34, and showed a similar fat accumulation inhibitory effect at 2.5 uM, which was a significantly lower concentration than olaparib 5 uM (FIG. 6), thereby identifying that JPI-289 had a significant effect compared to other PARP inhibitors.

### Example 4. Identification of NASH Inhibition Mechanism of JPI-289

### 4-1. In vitro HIF-1 and vimentin inhibitory effect

A MOA study was conducted to identify the mechanism that has a more significant effect than other PARP inhibitors as in Example 3-1. Specifically, the Hep3B cell line was treated with 300 uM H₂O₂ for 2 hours to generate a ROS rich situation for NASH, and treated with TCDD (Tetrachlorodibenzo-p-dioxin) at 10 nM for 24 hours to induce overexpression of TIPARP. Then, PARylation was identified, and the influences of JPI-289 (10 uM and 24 hours) and PJ34 (10 uM and 24 hours) were identified, respectively. In addition, DMOG, which induces hypoxia by inhibiting the degradation of HIF-1α, a hypoxia-related gene that appears in the early stages of fibrosis, was treated at 1 mM for 24 hours, and the influences of JPI-289 (10 uM and 24 hours) and PJ34 (10 uM and 24 hours) were also identified. In addition, as a positive control group, it was compared with INT-747, a drug that successfully completed phase 3 clinical trials for NASH indication.

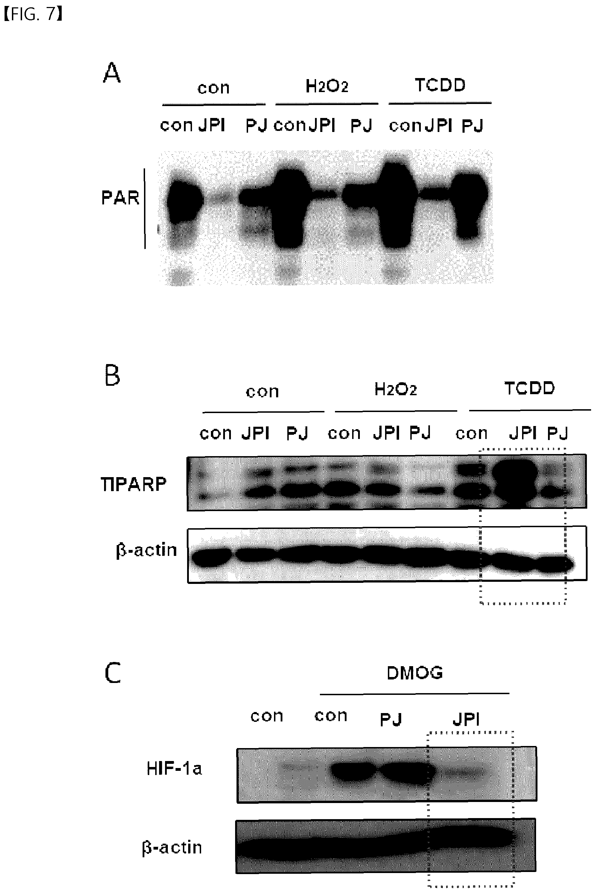

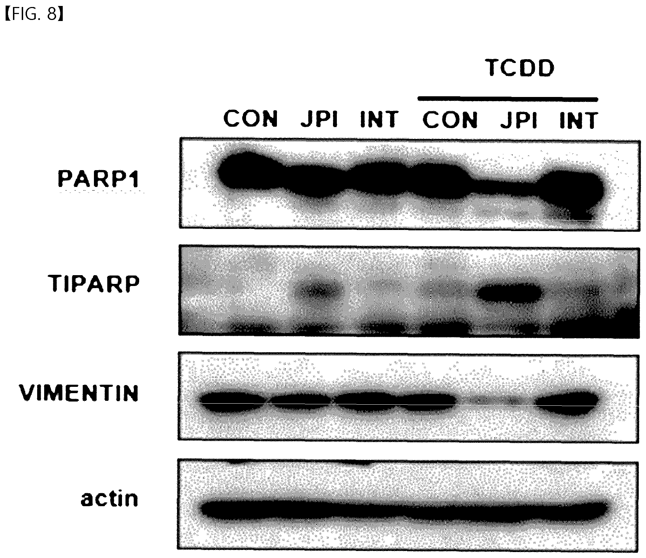

As a result, it was shown that JPI-289 significantly inhibited PARylation compared to PJ-34 (FIG. 7A). Upon TCDD administration, there was almost no increase in TIPARP in the PJ34 administration group, but the expression of TIPARP was significantly increased in the JPI-289 administration group (FIG. 7B). In addition, DMOG administration did not decrease the expression of HIF-1α in the PJ34 administration group, but significantly decreased the expression of HIF-1α in the JPI-289 administration group (FIG. 7C), thus identifying its effect on NASH and fibrosis. In addition, upon TCDD administration, the expression of PARP1 was significantly decreased, TIPARP was increased, and the expression of vimentin, a fibrosis marker, was also decreased in the JPI-289-treated group compared to the INT-747-treated group (FIG. 8).

### 4-2. In vivo HIF-1 inhibitory effect

To identify the HIF-1 inhibitory effect of INT-747 and JPI-289 in Example 4-1 also *in vivo*, 5-week-old mice were placed on a high-fat diet (HFD) 1 week after introduction. After 6 weeks, JPI-289 (50 mpk), PJ34 (10 mpk), and INT-747 (10 mpk) were administered for a total of 6 weeks, respectively, and the level of HIF-1α expression in the liver was identified by tissue immunochemical analysis.

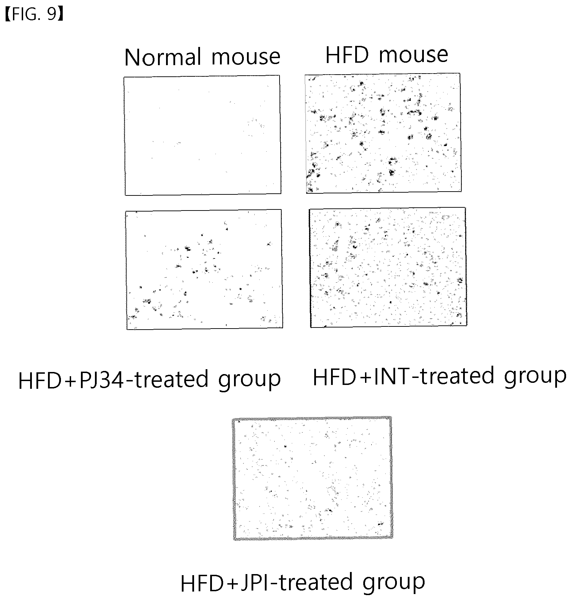

As a result, in the high-fat diet model, HIF-1α was significantly increased compared to normal mice, but in the group administered JPI-289, HIF-1α was significantly decreased compared to the group administered PJ34 or INT-747 (FIG. 9).

### 4-3. TGF-beta 2 inhibitory effect

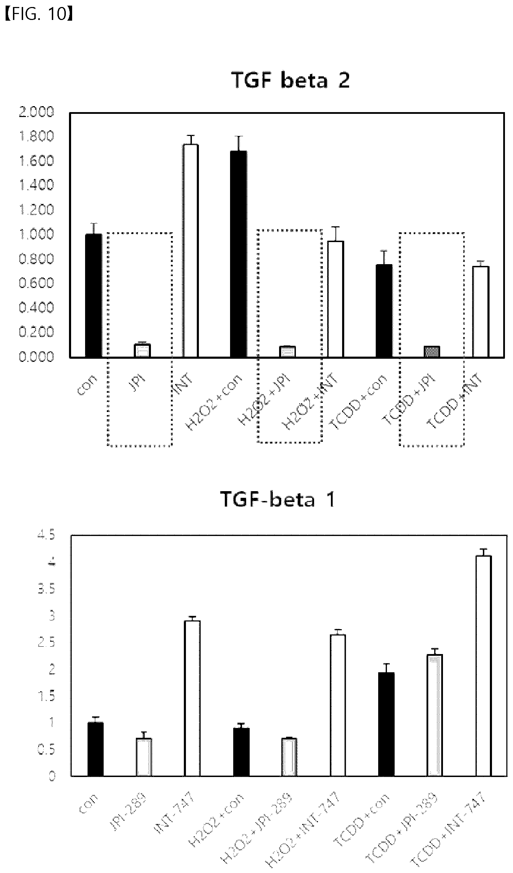

The inhibitory effects of TGF-beta2 (a downstream signal of HIF-1) by JPI-289 and INT-747 in Hep3B cell line were compared at the RNA level under the same conditions as in Example 4-1. As a result, it was shown that JPI-289, unlike TGF-beta1, significantly inhibited TGF-beta2 compared to INT-747 (FIG. 10).

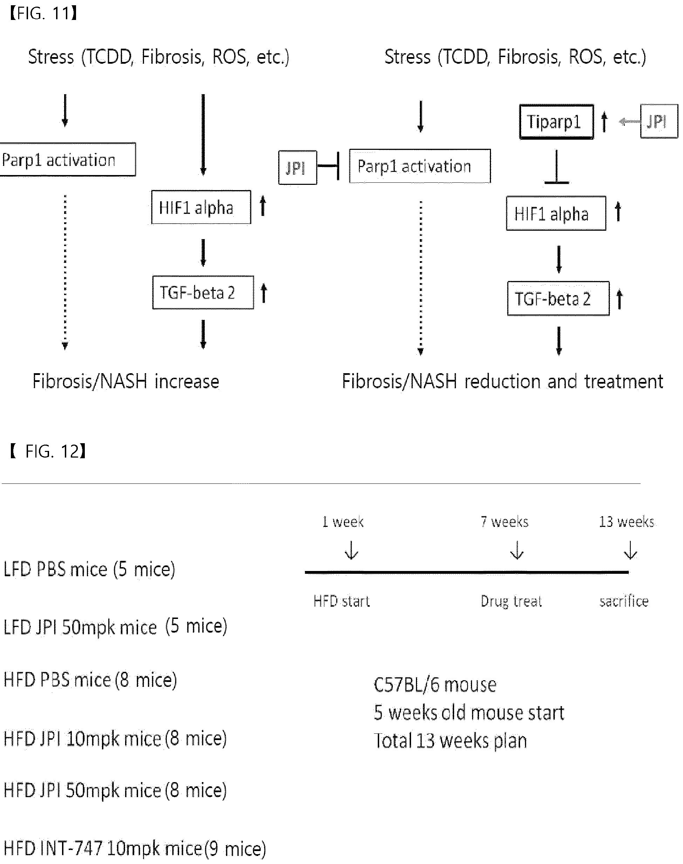

Thus, JPI-289 inhibited the activation of PARP1, which is related to NASH and NASH-induced fibrosis, and increased the expression of Tiparp, thereby inhibiting the expression of HIF-1α and its downstream signal, TGF-beta2, which are related to fibrosis, to inhibit NASH and NASH-induced fibrosis (FIG. 11).

### Example 5. In vivo Fat Accumulation Inhibitory Effect of JPI-289

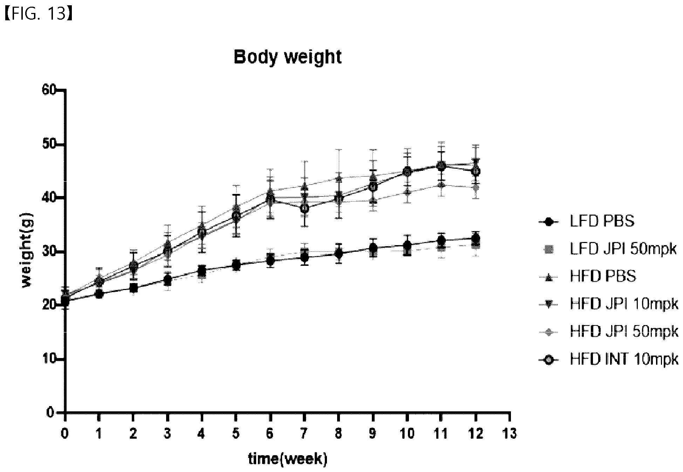

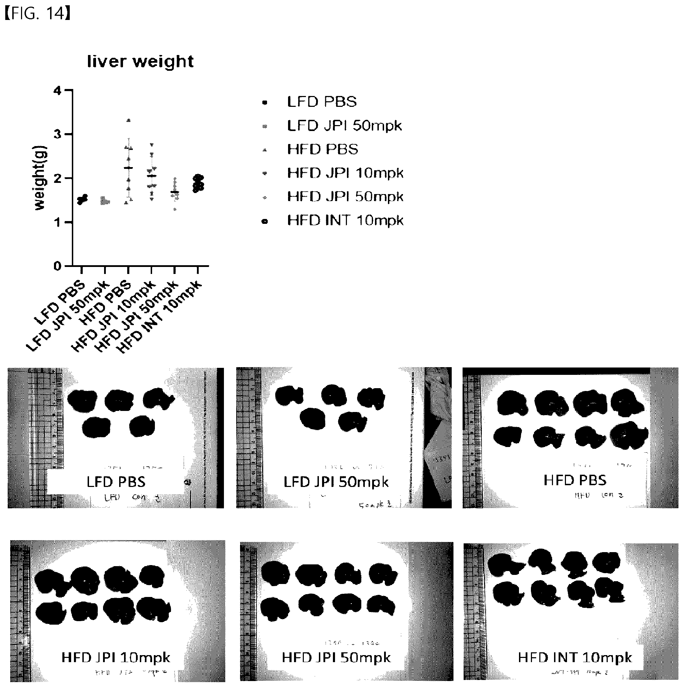

A high-fat diet animal model was created, and after 6 weeks, JPI-289 (JPI) and a positive control group of INT-747 (INT) were administered for a total of 6 weeks (FIG. 12), and body weight, liver size, and weight were checked. As a result, weight loss was identified in the group administered 50 mpk of JPI-289 compared to the high-fat diet group and the INT-747 administration group (FIG. 13). In addition, compared to the high-fat diet group, liver size and weight were decreased in the JPI-289 10 mpk administration group, JPI-289 50 mpk administration group, and INT-747 10 mpk administration group. In particular, in the JPI-289 50 mpk administration group, a significant decrease in liver size and weight was observed, which was more significant than that in the INT-747 group (FIG. 14).

### Example 6. In vivo NASH Treatment Effect by Short-Term Administration of JPI-289

### 6-1. NASH-induced fibrosis inhibitory effect

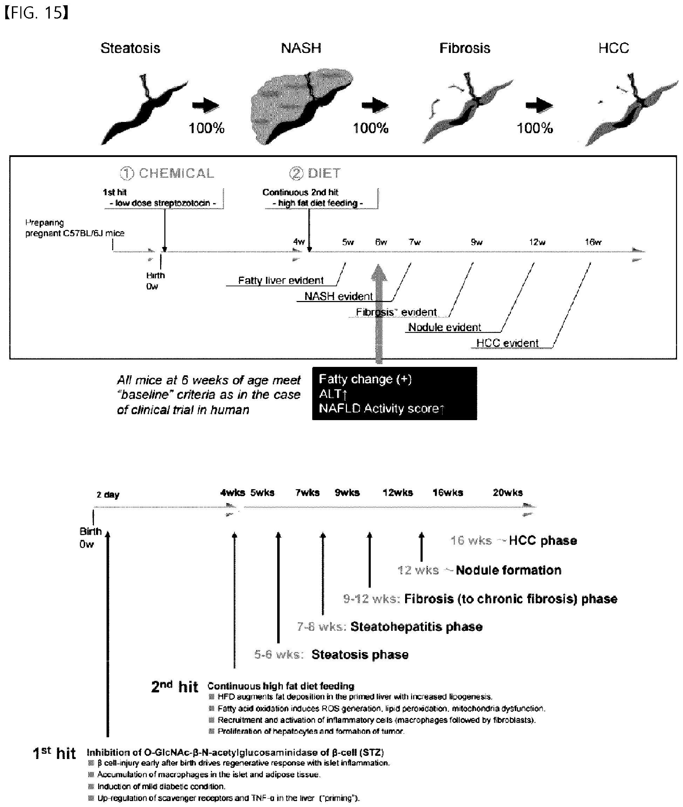

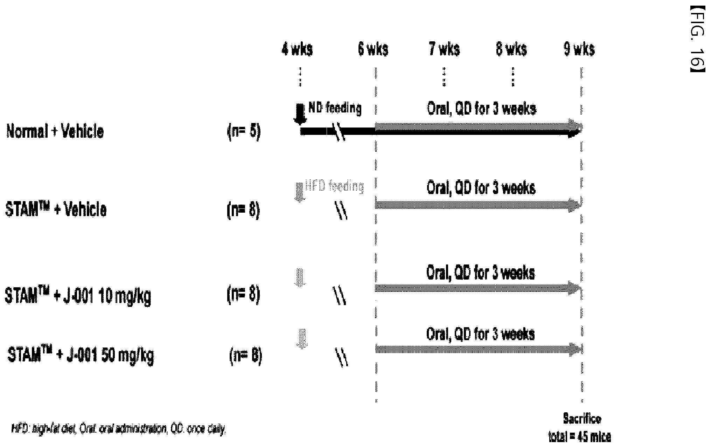

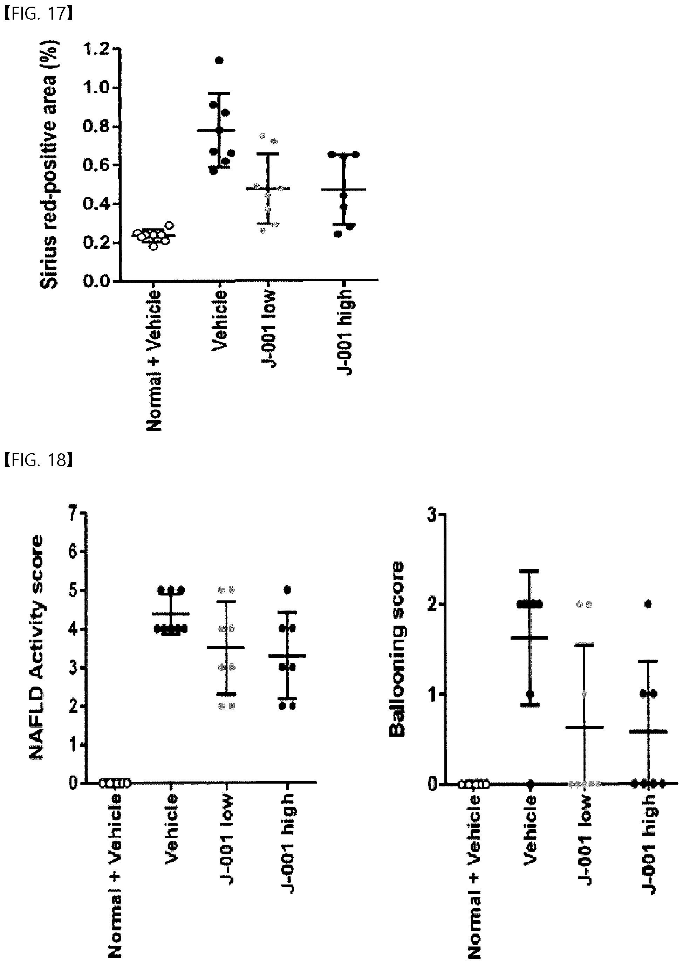

Streptozotocin was administered on the second day after birth, and a high-fat diet was administered starting 4 weeks after birth, causing non-alcoholic steatohepatitis (NASH) at 7 to 8 weeks of age. To identify the effect of JPI-289 on NASH and early fibrosis in the STAM^{™} mouse model (FIG. 15) in which fibrosis is induced by steatohepatitis at 9 to 12 weeks, the drug was administered at 10 mg/kg and 50 mg/kg, respectively for a total of 3 weeks, from 6 to 9 weeks, and the fibrosis-induced area was identified (FIG. 16). As a result, the fibrosis area was shown to be significantly decreased in all PI-289 administration groups (FIG. 17).

### 6-2. Fatty liver inhibitory effect

JPI-289 was administered as in Example 6-1, and the NAFLD score and ballooning score were identified as values for evaluating fatty liver. As a result, the NAFLD score was found to decrease in the JPI-289 administration group, and the ballooning score was also found to decrease (FIG. 18).

When administered for 3 weeks, JPI-289 was more effective than the control group in all dose groups, and the difference in effect depending on dose was not significant.

### Example 7. In vivo NASH Treatment Effect by Long-Term Administration of JPI-289

### 7-1. NASH-induced fibrosis inhibitory effect

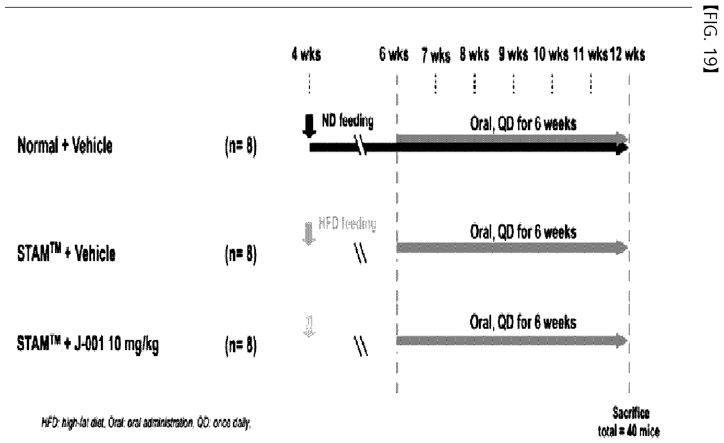

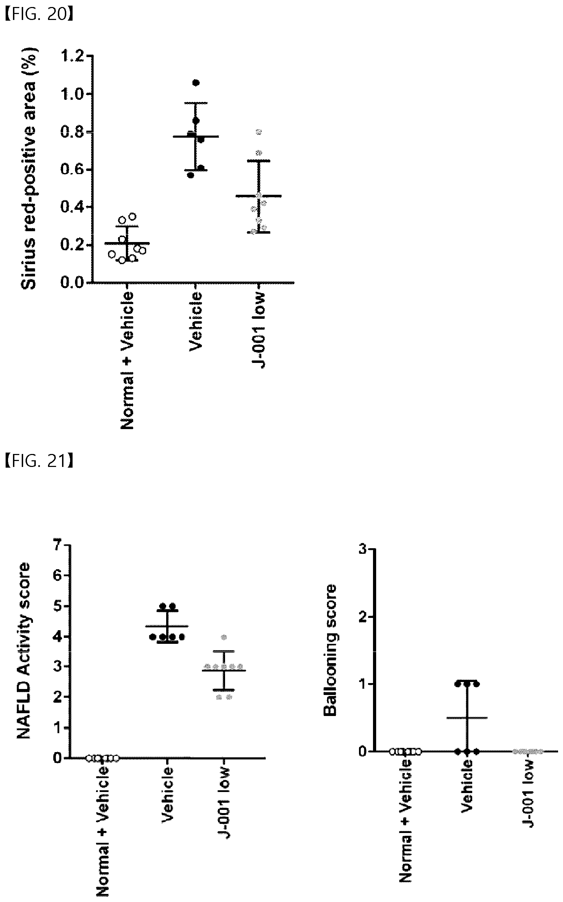

After administering JPI-289 (10 mg/kg) for a total of 6 weeks from 6 to 12 weeks in the same manner as in Example 6 (FIG. 19), the fibrosis area was checked. As a result, compared to the control group (Vehicle), the fibrosis area was significantly decreased in the JPI-289 10 mg/kg administration group (FIG. 20).

### 7-2. Fatty liver inhibitory effect

After administering JPI-289 (10 mg/kg) for a total of 6 weeks from 6 to 12 weeks in the same manner as in Example 6, the NAFLD score and ballooning score were checked to identify the degree of inhibition of fatty liver. As a result, compared to the control group, the NAFLD score was significantly decreased in the JPI-289 10 mg/kg administration group, and the ballooning score was also decreased in the JPI-289 10 mg/kg administration group compared to the control group (FIG. 21).

The group administered a low dose of JPI-289 for 6 weeks showed superior effect compared to the control group.

## Claims

1. A pharmaceutical composition for preventing or treating metabolic diseases, the pharmaceutical composition comprising, as an active ingredient, 10-ethoxy-8-(morpholinomethyl)-2,3,4,6-tetrahydrobenzo[h][1,6]naphthyridin-5(1H)-one, a pharmaceutically acceptable salt thereof, a hydrate thereof, or a salt hydrate thereof.

2. The pharmaceutical composition of claim 1, wherein the pharmaceutical composition contains a compound represented by Formula 1 below as an active ingredient: 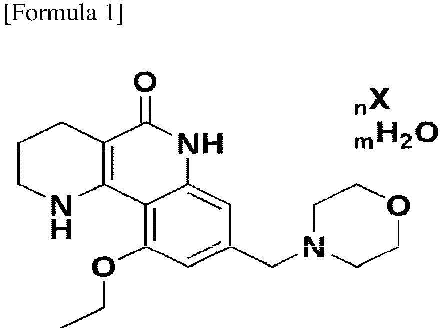 wherein, n or m is an integer from 0 to 3; and X is a pharmaceutically acceptable inorganic acid or organic acid.

3. The pharmaceutical composition of claim 2, wherein the n or m in Formula 1 is an integer from 0 to 3; and the X is one type selected from the group consisting of a hydrochloric acid, a benzene sulfonic acid, a maleic acid, a dimethane sulfonic acid, bis[7,7-dimethyl-2-oxobycyclo[2,2,1]heptane-1-yl)methanesulfonic acid], a tartaric acid, a 2,6-dioxo-1,2,3,6-tetrahydropyrimidine-4-carboxylic acid, an adipic acid, a nitrous acid (dinitric acid), a fumaric acid, a (S)-2-aminosuccinic acid, a 2-hydroxypropane-1,2,3-tricarboxylic acid, a cyclohexylsulphamic acid, a sulfuric acid, a succinic acid, a formic acid, a glutamic acid, and a diphosphoric acid.

4. The pharmaceutical composition of claim 1, wherein the pharmaceutical composition contains 10-ethoxy-8-(morpholinomethyl)-2,3,4,6-tetrahydrobenzo[h][1,6]naphthyridin-5(1H)-one dihydrochloride dihydrate as an active ingredient.

5. The pharmaceutical composition of claim 4, wherein the 10-ethoxy-8-(morpholinomethyl)-2,3,4,6-tetrahydrobenzo[h] [1,6]naphthyridin-5(1H)-one dihydrochloride dihydrate is a compound represented by Formula 2 below:

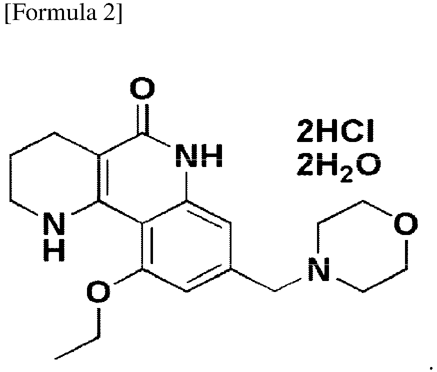

6. The pharmaceutical composition of claim 1, wherein the metabolic disease is any one selected from the group consisting of obesity, cirrhosis, and metabolic liver disease.

7. The pharmaceutical composition of claim 6, wherein the metabolic liver disease is fatty liver or non-alcoholic fatty liver disease (NAFLD).

8. The pharmaceutical composition of claim 7, wherein the non-alcoholic fatty liver disease is any one or more selected from the group consisting of non-alcoholic steatohepatitis (NASH), non-alcoholic fatty liver (NAFL), NAFLD-related liver fibrosis, NASH-related liver fibrosis, NAFLD-related liver cancer (Hepatocellular carcinoma), and NASH-related liver cancer (Hepatocellular carcinoma).

9. The pharmaceutical composition of claim 1, wherein the pharmaceutical composition inhibits PARylation.

10. The pharmaceutical composition of claim 1, wherein the pharmaceutical composition increases an expression of TIPARP (TCDD-inducible poly [ADP-ribose] polymerase).

11. The pharmaceutical composition of claim 1, wherein the pharmaceutical composition decreases an expression of HIF-1α (hypoxia inducible factor-1α).

12. The pharmaceutical composition of claim 1, wherein the pharmaceutical composition decreases an expression of PARP1 (Poly [ADP-ribose] polymerase 1).

13. The pharmaceutical composition of claim 1, wherein the pharmaceutical composition decreases an expression of vimentin.

14. The pharmaceutical composition of claim 1, wherein the pharmaceutical composition decreases an expression of TGF-beta2.

15. The pharmaceutical composition of claim 1, wherein the pharmaceutical composition decreases fat accumulation by inhibiting differentiation into adipocytes.

16. The pharmaceutical composition of claim 1, wherein the pharmaceutical composition inhibits fibrosis of the liver.

17. A use of 10-ethoxy-8-(morpholinomethyl)-2,3,4,6-tetrahydrobenzo[h][1,6]naphthyridin-5(1H)-one, a pharmaceutically acceptable salt thereof, a hydrate thereof, or a salt hydrate thereof, for preventing or treating metabolic diseases.

18. A method for preventing or treating metabolic diseases, the method comprising administering 10-ethoxy-8-(morpholinomethyl)-2,3,4,6-tetrahydrobenzo[h][1,6]naphthyridin-5(1H)-one, a pharmaceutically acceptable salt thereof, a hydrate thereof, or a salt hydrate thereof in a pharmaceutically effective amount to a subject suffering from the metabolic diseases.
